# EUROPEAN PATENT SPECIFICATION

(11) **EP 2 400 301 B1**
(45) Date of publication and mention of the grant of the patent: **05.06.2013**
(21) Application number: 10743598.4
(22) Date of filing: 23.02.2010
(51) Int. Cl.: G01N 33/53, G01N 33/543

(54) **ALLERGEN DETECTION METHOD USING IMMUNOCHROMATOGRAPHY**
ALLERGENERKENNUNGSVERFAHREN MITHILFE VON IMMUNCHROMATOGRAPHIE
MÉTHODE DE DÉTECTION DES ALLERGÈNES PAR IMMUNOCHROMATOGRAPHIE

(30) Priority: 23.02.2009 JP 2009039881; 27.03.2009 JP 2009079540
(43) Date of publication of application: 28.12.2011
(73) Proprietor: Prima Meat Packers, Ltd., Tokyo 140-8529 (JP)
(72) Inventor: KATO, Shigeki, Tsuchiura-shi Ibaraki 300-0841 (JP); AKIMOTO, Masanobu, Tsuchiura-shi Ibaraki 300-0841 (JP)
(74) Representative: Mallalieu, Catherine Louise
(86) International application number: PCT/JP2010/001209
(87) International publication number: WO 2010/095469

(56) References cited:
- EP-A1- 1 731 907
- WO-A1-02/37099
- WO-A1-2009/069779
- JP-A- 4 262 796
- JP-A- 10 042 869
- JP-A- 2005 106 811
- JP-A- 2006 071 509
- JP-A- 2006 317 226
- JP-A- 2007 278 773
- TAKAHATA Y ET AL: "Development of rapid and simple diagnostic kits for food allergens by immunochromatography", JOURNAL OF ALLERGY AND CLINICAL IMMUNOLOGY, MOSBY, INC, US, vol. 113, no. 2 supplement, 1 February 2004 (2004-02-01), page S237, XP009158793, ISSN: 0091-6749

## Description

### Technical Field

The present invention relates to a method for detecting allergens by immunochromatography, capable of rapid detection by extracting the allergens from a test sample such as food containing various allergens using an anionic surfactant and a thiosulfate, efficiently extracting the allergens, whether in a denatured or native form, with an anionic surfactant and a thiosulfate or an anionic surfactant and a non-ionic surfactant and further suppressing non-specific reactions accompanying the disintegration of colloidal gold conjugated to an antibody, and an allergen detection kit for immunochromatography capable of being used for the same.

### Background Art

Due to various factors including reduced natural environments, exhaust gases from cars, factories, and the like, housing conditions, or changes in food habits, one out of three persons is now said to have some allergic disease. Particularly, food allergy is a harmful immune reaction caused by the ingestion of an allergy-inducing substance contained in a food (hereinafter referred to as a food allergen) and sets up dermatitis, asthma, gastrointestinal tract disturbance, anaphylactic shock, or the like. Patients with such food allergies have increased, which poses serious problems medically and in the food industry. These harms sometimes lead to death and therefore must be treated before happens. For that purpose, the need for the provision of information to consumers through indication is increased; the Joint FAO/WHO Committee on Food Standards agreed that food products containing 8 raw materials known as allergic substances are labeled to the effect that they contain the raw materials and concluded that the member nations should consider a labeling method suitable for regulations in each nation (June, 1999). In Japan, labeling methods for 24 items of food having track records of causing severe allergic symptoms were prescribed considering the degree and frequency of past health hazards and the like (in effect from April, 2002). Known foods causing allergy include eggs, cow's milks, meats, fishes, crustaceans and mollusks, cereals, pulses and nuts, fruits, vegetables, beer yeast, or gelatin.

The simple immunoassay method for detecting a substance to be detected consisting of a particular antigen or antibody using a specific antigen-antibody reaction to rapidly and easily detect the allergen is an aggregation method involving binding the substance to be detected in a preparation to an antibody or antigen sensitized to particles by immunoreaction and measuring the aggregated state of the particles generated by the binding, which is a method commonly used particularly in that it enables visual determination.

A radioimmunoassay, enzyme immunoassay or fluorescence immunoassay method is also adopted which involves binding a substance to be detected in a preparation to an antibody or antigen labeled with a marker consisting of a radioisotope, an enzyme, or a fluorescent substance by immunoreaction and measuring the bound marker. In these immunoassay methods, a competition-type reaction and a sandwich-type reaction are widely used. Among these, an immunochromatography method is known as a so-called "measurement method using the sandwich-type reaction" (see for example, Patent Document 1), and various allergen detection kits are commercially available which are characterized by simplicity and rapidity in addition to high specificity resulting from an antigen-antibody reaction.

Preparations applied to such an immunochromatography method include biological preparations and extracts from foods; however, some types of preparations produce a so-called non-specific reaction by which light coloration occurs at trapped sites despite the absence of any specimen, which has sometimes caused reduced accuracy in a test. Accordingly, a developing solvent preventing non-specific aggregation and non-specific reaction during measurement and thereby enabling measurement with high accuracy is proposed, **characterized in that** a phosphorylcholine group-containing polymer is contained in a concentration of 0.005 to 0.3 w/v% in a buffer solution, wherein the polymer has a number average molecular weight of 40,000 or more (see for example, Patent Document 2).

For the above immunoassay methods, cases have also been observed where the occurrence of denaturation or the like of protein due to heating or pressurization provides lower measurement results or makes measurement impossible. In the sandwich ELISA method described in Notice No. 0122001 of the Department of Food Safety of the Ministry of Health, Labour and Welfare, Japan as a testing method for specific raw materials, an extraction method using an extraction solution containing a denaturant and a reducing agent (2-mercaptoethanol) is adopted for the sufficient extraction of allergens from a heated test preparation. To this method is applied a conventionally utilized sample preparation method for SDS-polyacrylamide electrophoresis used for protein analysis, and a denaturant and a reducing agent has been considered to be indispensable for increasing the efficiency of extraction of allergens from a test preparation. The application of a denaturant and a reducing agent to an immunochromatography method does not enable an accurate test because many non-specific reactions are observed, which has become a problem in measuring a denatured protein.

The present applicants have proposed an immunochromatography method capable of rapidly detecting allergens with good accuracy by extracting the allergens using an extraction solution containing a denaturant and a reducing agent and suppressing non-specific reactions accompanying the disintegration of the colloidal gold even when the extraction is applied to the immunochromatography method (see for example, Patent Document 3). This method has been able to be dramatically improved in accuracy and simplicity because the allergens are sufficiently extracted from a heated test preparation and further has become capable of being tested by a simple immunochromatography method. However, the reducing agent to be used (2-mercaptoethanol) has a peculiar smell and has been designated as a toxic substance since July 1, 2008; thus, it has become difficult to simply use the agent in food manufacturing facilities and the like. Hence, there has been a need for safer and efficient extraction methods and an immunochromatography method enabling accurate testing without any non-specific reaction being observed when these extraction methods are applied to the immunochromatography method.

### Prior Art Documents

### Patent Documents

Patent Document 1: Japanese Patent Laid-Open No. 05-010950
Patent Document 2: Japanese Patent Laid-Open No. 2003-344406
Patent Document 3: Japanese Patent Laid-Open No. 2007-278773

### Summary of the Invention

### Object to be Solved by the Invention

An object of the present invention is to provide a method for detecting allergens by immunochromatography, capable of rapidly detecting the allergens with good accuracy by extracting the allergens from a test sample such as food containing the allergens using an anionic surfactant and a thiosulfate or an anionic surfactant and a non-ionic surfactant, efficiently extracting the allergens, whether in a denatured or native form, with an anionic surfactant and thiosulfate or an anionic surfactant and a non-ionic surfactant and further suppressing non-specific reactions accompanying the disintegration of colloidal gold conjugated to antibodies, and an allergen detection kit for immunochromatography capable of being used for the same.

### Means to Solve the Object

The present inventors have found that allergens, whether in a denatured or native form, can be efficiently extracted from a heated test preparation with an anionic surfactant and a thiosulfate or an anionic surfactant and a non-ionic surfactant without using 2-mercaptoethanol, which is considered to be indispensible for sufficiently extracting allergens from a heated test preparation, and further that the use of a developing solution containing fetal bovine serum (FBS) in an immunochromatography method capable of easy detection can solve the above problems, thereby accomplishing the present invention.

Thus, the present invention relates to (1) a method for detecting allergen by immunochromatography which immunochromatography comprises using:
a colloidal gold-labeled antibody in which a colloidal gold is bound to a monoclonal antibody against denatured and native allergen,
a development support wherein a monoclonal antibody against denatured and native allergen recognizing an epitope different from that recognized by the colloidal gold-labeled antibody is fixed at a predetermined position, and
a developing solution containing a measurement sample of denatured and native allergen extracted from a test sample such as food containing an allergen, using an anionic surfactant and thiosulfate or an anionic surfactant and a non-ionic surfactant;
performing development on the development support; and detecting the allergen based on the presence of deposition of the colloidal gold;
wherein a developing solution containing at least 10% by weight of fetal bovine serum (FBS) is used, (2) the method for detecting an allergen by immunochromatography according to (1), wherein a developing solution containing at least 30% by weigh of fetal bovine serum (FBS), is-used, (3) the method for detecting an allergen by immunochromatography according to (2), wherein a developing solution containing at least 50% by weight of fetal bovine serum (FBS) is used, (4) the method for detecting an allergen by immunochromatography according to any one of (1) to (3), wherein the monoclonal antibody against denatured and native allergen are 2 types of monoclonal antibodies specifically recognizing denatured and native allergens selected from αs1 casein as a major component of milk allergen, β-lactoglobulin as a major component of whey allergen, ovalbumin and ovomucoid as egg white allergens, gliadin as a main component of wheat allergen, proteins having molecular weights of 24 kDa and 76 kDa as main proteins of buckwheat, and Arah1 as a main protein of peanut, (5) method for detecting an allergen by immunochromatography according to any one of (1) to (4), wherein dodecyl sodium sulfate is used as anionic surfactant, and (6) the method for detecting an allergen by immunochromatography according to any one of (1) to (5), wherein Tween 20 is used as non-ionic surfactant.

The present invention also relates to (7) a kit for detecting allergen by immunochromatography comprising: a colloidal gold-labeled antibody support carrying a colloidal gold-labeled antibody in which a colloidal gold is bound to a monoclonal antibody against denatured and native allergen; a development support wherein a monoclonal antibody against denatured and native allergen recognizing an epitope different from that recognized by the colloidal gold-labeled antibody is fixed at a predetermined position; a buffer solution containing an anionic surfactant and thiosulfate or an anionic surfactant and a non-ionic surfactant for extracting denatured and native allergen from a test sample such as food containing an allergen; a carrier for the sample capable of carrying a measurement sample of denatured and native allergen extracted from the test sample such as food containing the allergen with the anionic surfactant and thiosulfate or the anionic surfactant and the non-ionic surfactant; and fetal bovine serum (FBS) or a developing solution containing fetal bovine serum (FBS), (8) the kit for detecting allergen by immunochromatography according to (7), wherein the monoclonal antibody against denatured and native allergen are 2 types of monoclonal antibodies specifically recognizing denatured and native allergens selected from αs1 casein as a major component of milk allergen, β-lactoglobulin as a major component of whey allergen, ovalbumin and ovomucoid as egg white allergens, gliadin as a main component of wheat allergen, proteins having molecular weights of 24 kDa and 76 kDa as main proteins of buckwheat, and Arah1 as a main protein of peanut, and (9) the kit for detecting allergen by immunochromatography according to (7) or (8), comprising dodecyl sodium sulfate as anionic surfactant.

### Effect of the Invention

The method for detecting allergen by immunochromatography according to the present invention can rapidly detect various allergens with good accuracy by suppressing non-specific reactions accompanying the disintegration of colloidal gold conjugated to antibodies even when using an anionic surfactant and a thiosulfate or an anionic surfactant and a non-ionic surfactant.

### Mode of Carrying Out the Invention

The method for detecting allergen by immunochromatography according to the present invention is not particularly limited, provided that it is a method using a developing solution containing at least 10% by weight of fetal bovine serum (FBS) in an immunochromatography method which employs: a colloidal gold-labeled antibody in which a colloidal gold is bound to a monoclonal antibody against denatured and native allergen, a development support wherein a monoclonal antibody against denatured and native allergen recognizing an epitope different from that recognized by the colloidal gold-labeled antibody is fixed at a predetermined position, and a developing solution containing a measurement sample of denatured and native allergen extracted from a test sample such as food containing an allergen, using an anionic surfactant and thiosulfate oran anionic surfactant and a non-ionic surfactant; performing development on the development support; and then detecting the allergens based on the presence of the deposition of the colloidal gold. However, the method preferably uses a developing solution containing 20 to 100% by weight of fetal bovine serum (FBS), more preferably uses a developing solution containing 30 to 100% by weight thereof, particularly preferably uses a developing solution containing 40 to 100% by weight thereof, and still more preferably uses a developing solution containing 50 to 100% by weight thereof.

The kit for detecting allergen by immunochromatography according to the present invention is not particularly limited, provided that it is a detection kit comprising: a colloidal gold-labeled antibody support carrying a colloidal gold-labeled antibody in which a colloidal gold is bound to a monoclonal antibody against denatured and native allergen; a development support wherein a monoclonal antibody against denatured and native allergen recognizing an epitope different from that recognized by the colloidal gold-labeled antibody is fixed at a predetermined position; a buffer solution containing an anionic surfactant and thiosulfate or an anionic surfactant and a non-ionic surfactant for extracting denatured and native allergen from a test sample such as food containing an allergen; a carrier for the sample capable of carrying a measurement sample of denatured and native allergen extracted from the test sample such as food containing the allergen with the anionic surfactant and thiosulfate or the anionic surfactant and the non-ionic surfactant; and fetal bovine serum (FBS) or a developing solution containing fetal bovine serum (FBS). However, the kit preferably comprises a developing solution containing at least 10% by weight of fetal bovine serum (FBS), preferably a developing solution, containing at least 20% by weight thereof, more preferably a developing solution containing at least 30% by weight thereof, particularly preferably a developing solution containing at least 40% by weight thereof, still more preferably a developing solution containing at least 50% by weight, for example, 50 to 100% by weight, thereof.

A fetal bovine serum (FBS) concentration in the developing solution of less than 10% by weight is not preferable because it tends to cause non-specific reactions. The developing solution may also be prepared by, if necessary, suspending or emulsifying or dissolving additives such as a different surfactant, a preservative and an inorganic salt as well as fetal bovine serum (FBS) in the buffer solution. The buffer solution preferably has a pH of 4 to 10, particularly a pH of 6 to 8 and can be preferably exemplified by a phosphate buffer solution (PBS) and Tris buffer solution.

A method for preparing a colloidal gold-labeled antibody in which a colloidal gold is bound to a monoclonal antibody is not particularly limited, including a known conventional method; however, examples thereof can include a method which involves adding a solution in which the monoclonal antibodies are dissolved in a 2 mM borate buffer solution (pH 9.0) to a colloidal gold solution adjusted to pH 9.0 with a 0.2 M potassium carbonate solution, reacting the resultant solution at room temperature for 30 minutes, then adding a 10% BSA solution thereto, and further reacting the resultant for 15 minutes, followed by centrifugation. The colloidal gold-labeled antibody support can be prepared by applying the colloidal gold-labeled antibodies prepared above, for example, to a glass wool conjugate pad, followed by drying.

The development support can be prepared by linearly applying a buffer solution containing monoclonal antibodies to the denatured and native allergens recognizing epitopes different from those recognized by the colloidal gold-labeled antibodies, for example, to a nitrocellulose membrane and drying the resultant, followed by blocking treatment.

In the buffer solution used in extracting denatured and native allergens to prepare measurement samples, examples of the anionic surfactant can include a higher alcohol sulfate ester salt, an alkylnaphthalenesulfonate, an alkyl benzene sulfonate, and an alkyl phosphate; specifically, dodecyl sodium sulfate (SDS) can be preferably exemplified. Examples of the thiosulfate can include sodium thiosulfate, potassium thiosulfate, and ammonium thiosulfate; specifically, sodium thiosulfate can be preferably exemplified. Examples of the non-ionic surfactant can include a polyoxyethylene alkyl ether, a polyoxyethylene alkyl phenyl ether, a polyoxyethylene fatty acid ester, a polyglyceryl fatty acid ester, a polyoxyethylene fatty acid amide, a polyoxyethylene alkylamine, and a sorbitan fatty acid ester; specifically, polyoxyethylene (20) sorbitan monolaurate (Tween 20) can be preferably exemplified. The anionic surfactant has a concentration of 0.1 to 2.0%, preferably 0.25% to 0.5%; the thiosulfate has a concentration of 0.1 to 5.0%, preferably 0.1% to 1.0%; and the non-ionic surfactant has a concentration of 0.01 to 1.0%, preferably 0.05 to 0.2%. The use of a buffer solution containing these concentration ranges of the anionic surfactant and the thiosulfate or the anionic surfactant and the non-ionic surfactant is preferable in that it increases the extraction efficiency and can suppress non-specific reactions.

The carrier for the sample capable of carrying measurement samples can be exemplified by a glass wool sample pad. Then, the sample carrier, the colloidal gold-labeled antibody support, and the development support can be sequentially connected, preferably followed by further connecting an absorber such as an absorbent pad for absorbing the developing solution to the other end of the development support, thereby making a test piece for immunochromatography measurement. Then, measurement samples are spotted on the sample carrier, which is then immersed in a developing solution containing fetal bovine serum. As a result, allergens in the measurement sample is moved by capillarity and the like and bound to the colloidal gold-labeled antibodies. The antigen-antibody complexes are further moved onto the development support by capillarity and the like and trapped at the predetermined positions at which are immobilized monoclonal antibodies to the denatured and native allergens recognizing epitopes different from those recognized by the colloidal gold-labeled antibodies. The allergens can be detected by the presence of colored lines appearing at the predetermined positions.

The monoclonal antibodies to the denatured and native allergens can be preferably exemplified by 2 types of monoclonal antibodies specifically recognizing denatured and native allergens selected from αsl casein as a major component of milk allergen, β-lactoglobulin as a major component of whey allergen, ovalbumin and ovomucoid as egg white allergens, gliadin as a main component of wheat allergen, proteins having molecular weights of 24 kDa and 76 kDa as main proteins of buckwheat, and Arah1 as a main protein of peanut.

More specific examples of the anti-αs1 casein monoclonal antibodies can include an anti-αs1 casein monoclonal antibody, Pas1CN1, produced by a hybridoma (FERM-BP-10263) and an anti-αs1 casein monoclonal antibody, Pas1CN2, produced by a hybridoma (FERM-BP-10264), and examples of the anti-β-lactoglobulin monoclonal antibodies can include an anti-β-lactoglobulin monoclonal antibody, PβLG3, produced by a hybridoma (FERM- BP-11237) and an anti-β-lactoglobulin monoclonal antibody, PβLG4, produced by a hybridoma (FERM-BP-11238); all of these monoclonal antibodies were prepared by the present inventors. The hybridoma (FERM-BP-10263) and the hybridoma (FERM-BP-10264) were deposited February 24, 2005 in International Patent Organism Depositary, National Institute of Advanced Industrial Science and Technology (address: Tsukuba Central 6, 1-1-1 Higashi, Tsukuba, Ibaraki, Japan), and the hybridoma (FERM- BP-11237) and the hybridoma (FERM-BP-11238) were deposited February 22, 2010 in International Patent Organism Depositary, National Institute of Advanced Industrial Science and Technology (address: Tsukuba Central 6, 1-1-1 Higashi, Tsukuba, Ibaraki, Japan).

An anti-ovalbumin monoclonal antibody, PDOA3, produced by a hybridoma (FERM- BP-11235) and an anti-ovalbumin antibody, PDOA4, produced by a hybridoma (FERM-BP-11236) can also be exemplified. Examples of the anti-ovomucoid monoclonal antibodies can include an anti-ovomucoid monoclonal antibody, PNOM1, produced by a hybridoma (FERM-BP-10279) and an anti-ovomucoid monoclonal antibody, PNOM2, produced by a hybridoma (FERM-BP-10280) and an anti-ovomucoid monoclonal antibody, PDOM1, produced by a hybridoma (FERM-BP-10277) and an anti-ovomucoid monoclonal antibody, PDOM2, produced by a hybridoma (FERM-BP-10278). The hybridoma (FERM- BP-11235) and the hybridoma (FERM- BP-11236) were deposited February 22, 2010 in International Patent Organism Depositary, National Institute of Advanced Industrial Science and Technology (address: Tsukuba Central 6, 1-1-1 Higashi, Tsukuba, Ibaraki, Japan), and the hybridoma (FERM-BP-10279), the hybridoma (FERM-BP-10280), the hybridoma (FERM-BP-10277), and the hybridoma (FERM-BP-10278) were deposited February 24, 2005 in International Patent Organism Depositary, National Institute of Advanced Industrial Science and Technology (address: Tsukuba Central 6, 1-1-1 Higashi, Tsukuba, Ibaraki, Japan).

Examples of the anti-wheat gliadin monoclonal antibodies can include an anti-wheat gliadin monoclonal antibody, PGL1, produced by a hybridoma (FERM-BP-10267) and an anti-wheat gliadin monoclonal antibody, PGL2, produced by a hybridoma (FERM-BP-10268). The hybridoma (FERM-BP-10267) and the hybridoma (FERM-BP-10268) were deposited February 24, 2005 in International Patent Organism Depositary, National Institute of Advanced Industrial Science and Technology (address: Tsukuba Central 6, 1-1-1 Higashi, Tsukuba, Ibaraki, Japan).

Examples of the anti-buckwheat protein antibodies can include an anti-24 kDa protein monoclonal antibody, PBW5, produced by a hybridoma (FERM- BP-11241) and an anti-76 kDa protein monoclonal antibody, PBW2, produced by a hybridoma (FERM-BP-10273). The hybridoma (FERM- BP-11241) was deposited February 22, 2010 in International Patent Organism Depositary, National Institute of Advanced Industrial Science and Technology (address: Tsukuba Central 6, 1-1-1 Higashi, Tsukuba, Ibaraki, Japan), and the hybridoma (FERM-BP-10273) was deposited February 24, 2005 in International Patent Organism Depositary, National Institute of Advanced Industrial Science and Technology (address: Tsukuba Central 6, 1-1-1 Higashi, Tsukuba, Ibaraki, Japan).

Examples of the anti-peanut Arah1 protein monoclonal antibodies can include an anti-native Arah1 protein monoclonal antibody, PAh1-5, produced by a hybridoma (FERM- BP-11240) and an anti-native Arah1 protein monoclonal antibody, PAh1-4, produced by a hybridoma (FERM- BP-11239). The hybridoma (FERM- BP-11240) and the hybridoma (FERM- BP-11239) were deposited February 22, 2010 in International Patent Organism Depositary, National Institute of Advanced Industrial Science and Technology (address: Tsukuba Central 6, 1-1-1 Higashi, Tsukuba, Ibaraki, Japan).

The present invention will be more specifically described below with reference to Examples. However, these Examples are not intended to limit the technical scope of the present invention.

### Examples

### Example 1

### [Detection of Ovalbumin Extracted with Anionic Surfactant and Thiosulfate by Immunochromatography]

### 1. Materials and Methods

### (1) Preparation of Colloidal Gold-Labeled Antibody

A MAb solution of PDOA4 (FERM-BP-11236) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M potassium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of PDOA3 (FERM- BP-11235) was prepared to have a concentration of 4 mg/ml in PBS and linearly applied to a nitrocellulose membrane, followed by drying. Then, the resultant was subjected to blocking using TBS containing 1% skim milk at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The following model meat products were tested as samples for detection.

### 1) Preparation of Egg Protein

An egg protein was prepared from commercial hen's eggs according to Akiyama et al. (Inter-laboratory Evaluation Studies of Notified ELISA Methods for Allergic Substances (Egg). J. Food hyg. Soc. Japan, 44, 2003, 213-219).

### 2) Model Meat Product

A meat product was selected as a model food for a quantitative test, and model meat products each containing an egg protein with the concentration in the formulation shown in Table 1 were prepared. As a porcine red meat was used a porcine loin meat from which a line meat was removed and which was ground in 5 mm diameter pieces. Additives are weighed according to each formulation, mixed using a food processor, and then packed in a vinyl chloride tube.

**[Table 1]**

| <Table of Formulation for Model Meat Product (Egg)> | | | |
|---|---|---|---|
| Raw Material | Egg 10ppm | Egg 2ppm | Control |
| Porcine Red Meat (%) | 83.0 | 83.0 | 83.0 |
| NaCl (%) | 2.0 | 2.0 | 2.0 |
| Na Polyphosphate (%) | 0.2 | 0.2 | 0.2 |
| Sodium Nitrite (ppm) | 120 | 120 | 120 |
| Sodium Ascorbate (ppm) | 300 | 300 | 300 |
| Water (%) | 14.5 | 14.5 | 14.5 |
| Egg Protein (ppm) | 10 | 2 | 0 |
| Total (%) | 99.744 | 99.7422 | 99.742 |

### 3) Heating Temperature and Time

The mixture was heated at 75°C for 30 minutes and then homogenized using a food processor, and the resultant was used as a sample for detection.

### 4) Pretreatment of Sample

1 g of the sample for detection was weighed, and 19 ml of PBS containing 0.5% SDS and a final concentration of 0% to 10.0% of sodium thiosulfate was added thereto as an extraction solution. The resultant was stirred, heated in boiling water for 1 hour, and cool centrifuged, and the supernatant was then used as a measurement sample.

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection.

### 2. Result

Then, the detection results are shown in Table 2. The determination was expressed by +, +w or +- in order of decreasing strength of lines, and negativity was expressed as -.

**[Table 2]**

| <Test Result of Model Meat Product Heated at 75°C for 30 Minutes (Egg)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | + | + | + | - |
| 2ppm | +- | +W | +W | +W | +W | +- | - |
| 0ppm | - | - | - | - | - | - | - |

As shown in Table 2, when the concentration of sodium thiosulfate was 0% to 5.0%, the protein was detected down to 2 ppm, at which the determination was made as +w at 0.1% to 2.0% and the visibility was best in this range. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 2

### [Detection of Ovalbumin Extracted with Anionic Surfactant and Non-Ionic Surfactant by Immunochromatography]

### 1. Materials and Methods

1) Detection was performed in the same way as in Example 1 except for the following "heating temperature and time", "pretreatment of sample" and "confirmation of detection by immunocromatography".
2) Heating Temperature and Time
   The mixture was heated at 75°C for 30 minutes or 121°C for 20 minutes.
3) After heating, the resultant was homogenized using a food processor, and used as a sample for detection. 1 g of the sample for detection was weighed, and 19 ml of PBS containing 0.5% SDS was added thereto. The resultant was stirred, heated in boiling water for 1 hour, and cool centrifuged, and the supernatant was then used as a measurement sample [1]. Tween 20 was added to the measurement sample [1] so as to provide a final concentration of 0.2% to make a measurement sample [2]. In addition, 19 ml of PBS containing 0.5% SDS and 0.2% Tween 20 was added thereto, which was then stirred, heated in boiling water for 1 hour, and cool centrifuged, and the supernatant was then used as a measurement sample [3]. The measurement sample [2] is to examine whether the presence of Tween 20 is involved in the sensitivity of the immunochromatography kit in measuring the sample using the immunochromatography kit because Tween 20 is added thereto after SDS extraction and is not involved in extraction. The measurement sample [3] is for examining whether Tween 20 contributes to the extraction efficiency because the extraction is performed by the presence of both SDS and Tween 20.
4) Confirmation of Detection by Immunochromatography 20 µl of the prepared colloidal gold-labeled antibody, 30 µl of fetal bovine serum (FBS) as a developing solution, and 50 µl of the measurement sample [1], the measurement sample [2] or the measurement sample [3] were added, which was tested on an immunochromatographic strip for confirmation of detection.

### 2. Result

Then, the detection results are shown in Tables 3 and 4. The determination was expressed by +, +w or +- in order of decreasing strength of lines, and negativity was expressed as -.

**[Table 3]**

| <Test Result of Model Meat Product Heated at 75°C for 30 Minutes (Egg)> | | | |
|---|---|---|---|
| 75°C for 30 Minutes | Model Meat Product | | |
| | 10ppm | 2ppm | 0ppm |
| Measurement Sample [1] | + | + | - |
| Measurement Sample [2] | + | + | - |
| Measurement Sample [3] | + | + | - |

**[Table 4]**

| <Test Result of Model Meat Product Heated at 121°C for 20 Minutes (Egg)> | | | |
|---|---|---|---|
| 121°C for 20 Minutes | Model Meat Product | | |
| | 10ppm | 2ppm | 0ppm |
| Measurement Sample [1] | + | - | - |
| Measurement Sample [2] | +W | - | - |
| Measurement Sample [3] | + | +W | - |

As shown in Table 3, for the model meat product heated at 75°C for 30 minutes, the determination was made as + down to 2 ppm in all measurement samples; no non-specific reactions were observed at 0 ppm. On the other hand, as shown in Table 4, for the model meat product heated at 121°C for 20 minutes, the determination was made as +w down to 2 ppm in the measurement sample [3], indicating that the egg protein was able to be detected down to 2 ppm. No non-specific reactions were observed at 0 ppm. This showed that an extraction method in which an anionic surfactant and a non-ionic surfactant were combined was high in the extraction efficiency and eliminated non-specific reactions by using FBS as a developing solution, enabling the construction of a rapid immunochromatography kit with good accuracy.

### Example 3

### [Detection of Casein Extracted with Anionic Surfactant and Thiosulfate by Immunochromatography]

### 1. Materials and Methods

### (1) Preparation of Colloidal Gold-Labeled Antibody

A MAb solution of Pαs1CN2 (FERM-BP-10264) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M potassium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of Pαs1CN1(FERM-BP-10263) was prepared to have a concentration of 4 mg/ml in PBS and linearly applied to a nitrocellulose membrane, followed by drying. Then, the resultant was subjected to blocking using TBS containing 0.1% bovine hide gelatin at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The milk protein was prepared from fresh holstein milk according to the method of Akiyama et al. The model meat products having the formulations shown in Table 5 were tested as samples for detection; the conditions of heating temperature and time and sample pretreatment were the same as those in Example 1.

**[Table 5]**

| <Table of Formulation for Model Meat Product (Milk)> | | | |
|---|---|---|---|
| Raw Material | Milk 10ppm | Milk 2ppm | Control |
| Porcine Red Meat (%) | 83.0 | 83.0 | 83.0 |
| NaCl (%) | 2.0 | 2.0 | 2.0 |
| Na Polyphosphate (%) | 0.2 | 0.2 | 0.2 |
| Sodium Nitrite (ppm) | 120 | 120 | 120 |
| Sodium Ascorbate (ppm) | 300 | 300 | 300 |
| Water (%) | 14.5 | 14.5 | 14.5 |
| Milk Protein (ppm) | 10 | 2 | 0 |
| Total (%) | 99.744 | 99.7422 | 99.742 |

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection

### 2. Result

Then, the detection results are shown in Table 6. The determination was expressed by +, +w or +- in order of decreasing strength of lines, and negativity was expressed as -.

**[Table 6]**

| <Test Result of Model Meat Product Heated at 75°C for 30 Minutes (Casein)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | + | + | +W | - |
| 2ppm | +- | +W | +W | +W | +- | - | - |
| 0ppm | - | - | - | - | - | - | - |

As shown in Table 6, when the concentration of sodium thiosulfate was 0% to 2.0%, the protein was detected down to 2 ppm, at which the determination was made as +w at 0.1% to 1.0% and the visibility was best in this range. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 4

### [Detection of Casein Extracted with Anionic Surfactant and Non-Ionic Surfactant by Immunochromatography]

### 1. Materials and Methods

1) Detection was performed in the same way as in Example 3 except that the conditions of the temperature and time of heating model meat products, the pretreatment of samples and the confirmation of detection by immunochromatography were the same as those in Example 2.

### 2. Result

Then, the detection results are shown in Tables 7 and 8. The determination was expressed by +, +w or +- in order of decreasing strength of lines, and negativity was expressed as -.

**[Table 7]**

| <Test Result of Model Meat Product Heated at 75°C for 30 Minutes (Casein) | | | |
|---|---|---|---|
| 75°C for 30 Minutes | Model Meat Product | | |
| | 10ppm | 2ppm | 0ppm |
| Measurement Sample [1] | + | +- | - |
| Measurement Sample [2] | + | +- | - |
| Measurement Sample [3] | + | +W | - |

**[Table 8]**

| <Test Result of Model Meat Product Heated at 121°C for 20 Minutes (Casein)> | | | |
|---|---|---|---|
| 121°C for 20 Minutes | Model Meat Product | | |
| | 10ppm | 2ppm | 0ppm |
| Measurement Sample [1] | + | +- | - |
| Measurement Sample [2] | + | +- | - |
| Measurement Sample [3] | + | +W | - |

As shown in Tables 7 and 8, the determination was made as +w down to 2 ppm in the measurement sample [3], indicating that the milk protein was able to be detected down to 2 ppm. No non-specific reactions were observed at 0 ppm. This showed that as seen for the measurement sample [3], an extraction method in which an anionic surfactant and a non-ionic surfactant were combined was the highest in the extraction efficiency and eliminated non-specific reactions by using FBS as a developing solution, enabling the construction of a rapid immunochromatography kit with good accuracy.

### Example 5

### [Detection of Whey Extracted with Anionic Surfactant and Thiosulfate by Immunochromatography]

### 1. Materials and Methods

### (1) Preparation of Colloidal Gold-Labeled Antibody

A MAb solution of PβLG4 (FERM- BP-11238) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M potassium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of PβLG3 (FERM-BP-11237) was prepared to have a concentration of 4 mg/ml in PBS and linearly applied to a nitrocellulose membrane, followed by drying. Then, the resultant was subjected to blocking using TBS containing 0.1% bovine hide gelatin at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The milk protein was prepared from fresh holstein milk according to the method of Akiyama et al. The model meat products having the formulations shown in Table 3 were tested as samples for detection; the conditions of heating temperature and time and sample pretreatment were the same as those described above.

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection.

### 2. Result

Then, the detection results are shown in Table 9. The determination was expressed by +, +w or +- in order of decreasing strength of lines, and negativity was expressed as -.

**[Table 9]**

| <Test Result of Model Meat Product Heated at 75°C for 30 Minutes (Whey)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | +W | +- | - | - |
| 2ppm | +W | +W | +- | +- | - | - | - |
| 0ppm | - | - | - | - | - | - | - |

As shown in Table 9, when the concentration of sodium thiosulfate was 0% to 1.0%, the protein was detected down to 2 ppm and the visibility was best at 0.1%. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 6

### [Detection of Whey Extracted with Anionic Surfactant and Non-Ionic Surfactant by Immunochromatography]

### 1. Materials and Methods

1) Detection was performed in the same way as in Example 5 except that the conditions of the temperature and time of heating model meat products, the pretreatment of samples and the confirmation of detection by immunochromatography were the same as those in Example 2.

### 2. Result

Then, the detection results are shown in Tables 10 and 11. The determination was expressed by +, +w or +- in order of decreasing strength of lines, and negativity was expressed as -.

**[Table 10]**

| <Test Result of Model Meat Product Heated at 75°C for 30 Minutes (Whey)> | | | |
|---|---|---|---|
| 75°C for 30 Minutes | Model Meat Product | | |
| | 10ppm | 2ppm | 0ppm |
| Measurement Sample [1] | + | +W | - |
| Measurement Sample [2] | + | +W | - |
| Measurement Sample [3] | + | +W | - |

**[Table 11]**

| <Test Result of Model Meat Product Heated at 121°C for 20 Minutes (Whey)> | | | |
|---|---|---|---|
| 121°C for 20 Minutes | Model Meat Product | | |
| | 10ppm | 2ppm | 0ppm |
| Measurement Sample [1] | + | - | - |
| Measurement Sample [2] | + | - | - |
| Measurement Sample [3] | + | - | - |

As shown in Table 10, the determination was made as +w down to 2 ppm in all measurement samples, indicating that the milk protein was able to be detected down to 2 ppm. No non-specific reactions were observed at 0 ppm. As shown in Table 11, the protein was able to be detected as + at 10 ppm in all measurement samples; however, as in the measurement sample [3], the visibility was best with the extraction method in which the anionic surfactant and the non-ionic surfactant were combined. Thus, it was shown that an extraction method in which an anionic surfactant and a non-ionic surfactant were combined was high in the extraction efficiency and eliminated non-specific reactions by using FBS as a developing solution, enabling the construction of a rapid immunochromatography kit with good accuracy.

### Example 7

### [Detection of Wheat Protein Extracted with Anionic Surfactant and Thiosulfate by Immunochromatography]

### 1. Materials and Methods

### (1) Preparation of Colloidal Gold-Labeled Antibody

A MAb solution of PGL2 (FERM-BP-10268) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M sodium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of PGL1 (FERM-BP-10267) was prepared to have a concentration of 4 mg/ml in PBS and linearly applied to a nitrocellulose membrane, followed by drying. Then, the resultant was subjected to blocking using TBS containing 1.0% bovine hide gelatin at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The wheat protein was prepared from commercial wheat powder according to the method of Akiyama et al. The model meat products having the formulations shown in Table 12 were tested as samples for detection; the conditions of heating temperature and time and sample pretreatment were the same as those described above.

**[Table 12]**

| <Table of Formulation for Model Meat Product (Wheat)> | | | |
|---|---|---|---|
| Raw Material | Wheat 10ppm | Wheat 2ppm | Control |
| Porcine Red Meat (%) | 83.0 | 83.0 | 83.0 |
| NaCl (%) | 2.0 | 2.0 | 2.0 |
| Na Polyphosphate (%) | 0.2 | 0.2 | 0.2 |
| Sodium Nitrite (ppm) | 120 | 120 | 120 |
| Sodium Ascorbate (ppm) | 300 | 300 | 300 |
| Water (%) | 14.5 | 14.5 | 14.5 |
| Wheat Protein (ppm) | 10 | 2 | 0 |
| Total (%) | 99.744 | 99.7422 | 99.742 |

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection.

### 2. Result

Then, the detection results are shown in Table 13. The determination was expressed by +, +w or +- in order of decreasing strength of lines, and negativity was expressed as -.

**[Table 13]**

| <Test Result of Model Meat Product Heated at 75°C for 30 Minutes (Wheat)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | + | + | + | +W |
| 2ppm | + | + | + | + | + | + | +- |
| 0ppm | - | - | - | - | - | - | - |

As shown in Table 13, when the concentration of sodium thiosulfate was 0% to 10.0%, the protein was detected down to 2 ppm, at which the determination was made as + at 0% to 5.0%; the visibility was best particularly at 0.1% to 2.0%. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 8

### [Detection of Wheat Protein Extracted with Anionic Surfactant and Non-Ionic Surfactant by Immunochromatography]

### 1. Materials and Methods

1) Detection was performed in the same way as in Example 7 except that the conditions of the temperature and time of heating model meat products, the pretreatment of samples and the confirmation of detection by immunochromatography were the same as those in Example 2.

### 2. Result

Then, the detection results are shown in Tables 14 and 15. The determination was expressed by +, +w or +- in order of decreasing strength of lines, and negativity was expressed as -.

**[Table 14]**

| <Test Result of Model Meat Product Heated at 75°C for 30 Minutes (Wheat)> | | | |
|---|---|---|---|
| 75°C for 30 Minutes | Model Meat Product | | |
| | 10ppm | 2ppm | 0ppm |
| Measurement Sample [1] | + | + | - |
| Measurement Sample [2] | + | + | - |
| Measurement Sample [3] | + | + | - |

**[Table 15]**

| <Test Result of Model Meat Product Heated at 121°C for 20 Minutes (Wheat)> | | | |
|---|---|---|---|
| 121°C for 20 Minutes | Model Meat Product | | |
| | 10ppm | 2ppm | 0ppm |
| Measurement Sample [1] | + | + | - |
| Measurement Sample [2] | + | + | - |
| Measurement Sample [3] | + | + | - |

As shown in Tables 14 and 15, the determination was made as + down to 2 ppm in all measurement samples, indicating that the wheat protein was able to be detected down to 2 ppm. No non-specific reactions were observed at 0 ppm. As in the measurement sample [3], the visibility was best with the extraction method in which the anionic surfactant and the non-ionic surfactant were combined. Thus, it was shown that an extraction method in which an anionic surfactant and a non-ionic surfactant were combined was high in the extraction efficiency and eliminated non-specific reactions by using FBS as a developing solution, enabling the construction of a rapid immunochromatography kit with good accuracy.

### Example 9

### [Detection of Buckwheat Protein Extracted with Anionic Surfactant and Thiosulfate by Immunochromatography]

### 1. Materials and Methods

### (1) Preparation of Colloidal Gold-Labeled Antibody

A MAb solution of PBW2 (FERM-BP-10273) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M potassium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of PBW5 (FERM- BP-11241) was prepared to have a concentration of 4 mg/ml in PBS and linearly applied to a nitrocellulose membrane, followed by drying. Then, the resultant was subjected to blocking using TBS containing 1% skim milk at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The buckwheat protein was prepared from commercial buckwheat powder according to the method of Akiyama et al. The model meat products having the formulations shown in Table 16 were tested as samples for detection; the conditions of heating temperature and time and sample pretreatment were the same as those described above.

**[Table 16]**

| <Table of Formulation for Model Meat Product (Buckwheat)> | | | |
|---|---|---|---|
| Raw Material | Buckwheat 10ppm | Buckwheat 2ppm | Control |
| Porcine Red Meat (%) | 83.0 | 83.0 | 83.0 |
| NaCl (%) | 2.0 | 2.0 | 2.0 |
| Na Polyphosphate (%) | 0.2 | 0.2 | 0.2 |
| Sodium Nitrite (ppm) | 120 | 120 | 120 |
| Sodium Ascorbate (ppm) | 300 | 300 | 300 |
| Water (%) | 14.5 | 14.5 | 14.5 |
| Buckwheat Protein (ppm) | 10 | 2 | 0 |
| Total (%) | 99.744 | 99.7422 | 99.742 |

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection.

### 2. Result

Then, the detection results are shown in Table 17. The determination was expressed by +, +w or +- in order of decreasing strength of lines, and negativity was expressed as -.

**[Table 17]**

| <Test Result of Model Meat Product Heated at 75°C for 30 Minutes (Buckwheat)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | + | + | + | +W |
| 2ppm | - | +- | +W | +- | +- | +W | +- |
| 0ppm | - | - | - | - | - | - | - |

As shown in Table 17, when the concentration of sodium thiosulfate was 0.1% to 5.0%, the protein was detected down to 2 ppm, at which the determination was made as +w particularly at 0.5% and 5.0% and the visibility was best in these concentrations. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 10

### [Detection of Buckwheat Protein Extracted with Anionic Surfactant and Non-Ionic Surfactant by Immunochromatography]

### 1. Materials and Methods

1) Detection was performed in the same way as in Example 9 except that the conditions of the temperature and time of heating model meat products, the pretreatment of samples and the confirmation of detection by immunochromatography were the same as those in Example 2.

### 2. Result

Then, the detection results are shown in Tables 18 and 19. The determination was expressed by +, +w or +- in order of decreasing strength of lines, and negativity was expressed as -.

**[Table 18]**

| <Test Result of Model Meat Product Heated at 75°C for 30 Minutes (Buckwheat)> | | | |
|---|---|---|---|
| 75°C for 30 Minutes | Model Meat Product | | |
| | 10ppm | 2ppm | 0ppm |
| Measurement Sample [1] | +- | - | - |
| Measurement Sample [2] | + | - | - |
| Measurement Sample [3] | + | +- | - |

**[Table 19]**

| <Test Result of Model Meat Product Heated at 121°C for 20 Minutes (Buckwheat)> | | | |
|---|---|---|---|
| 121°C for 20 Minutes | Model Meat Product | | |
| | 10ppm | 2ppm | 0ppm |
| Measurement Sample [1] | + | +- | - |
| Measurement Sample [2] | + | +- | - |
| Measurement Sample [3] | + | +W | - |

As shown in Table 18, for the model meat product heated at 75°C for 30 minutes, the determination was made as +- down to 2 ppm in the measurement sample [3]; no non-specific reactions were observed at 0 ppm. As shown in Table 19, for the model meat product heated at 121°C for 20 minutes, the determination was made as +w down to 2 ppm in the measurement sample [3], indicating that the buckwheat protein was able to be detected down to 2 ppm. No non-specific reactions were observed at 0 ppm. This showed that an extraction method in which an anionic surfactant and a non-ionic surfactant were combined was high in the extraction efficiency and eliminated non-specific reactions by using FBS as a developing solution, enabling the construction of a rapid immunochromatography kit with good accuracy.

### Example 11

### [Detection of Peanut Protein Extracted with Anionic Surfactant and Thiosulfate by Immunochromatography]

### - 1. Materials and Methods

### (1) Preparation of Colloidal Gold-Labeled Antibody

A MAb solution of PAh1-4 (FERM- BP-11239) was prepared to have a concentration of 1 mg/ml in a 2 mM borate buffer solution (pH 9.0). 500 µl of the MAb solution was added to 5 ml of a colloidal gold solution (from Sigma) pre-adjusted to pH 9.0 with a 0.2 M potassium carbonate solution, which was then reacted at room temperature for 30 minutes. Then, 635 µl of a 10% BSA solution was added thereto, which was further reacted for 15 minutes. Centrifugation was carried out, followed by adjustment to OD525 = 1.0 using a 1% BSA solution.

### (2) Preparation of Antibody-Immobilized Membrane

A MAb solution of PAh1-5 (FERM-BP-11240) was prepared to have a concentration of 4 mg/ml in PBS and linearly applied to a nitrocellulose membrane, followed by drying. Then, the resultant was subjected to blocking using TBS containing 1% skim milk at 37°C for 1 hour and then washed with TBS and dried.

### (3) Assembly of Immunochromatographic Strip

In addition to the antibody-immobilized membrane, a glass wool sample pad for spotting a test solution and a glass wool absorbent pad for absorbing the test solution were separately provided, and the sample pad, the antibody-immobilized membrane, and the absorbent pad were attached in that order to make an immunochromatographic strip. The buckwheat protein was prepared from defatted peanuts according to the method of Akiyama et al. The model meat products having the formulations shown in Table 20 were tested as samples for detection; the conditions of heating temperature and time and sample pretreatment were the same as those described above.

**[Table 20]**

| <Table of Formulation for Model Meat Product (Peanut)> | | | |
|---|---|---|---|
| Raw Material | Peanut 10ppm | Peanut 2ppm | Control |
| Porcine Red Meat (%) | 83.0 | 83.0 | 83.0 |
| NaCl (%) | 2.0 | 2.0 | 2.0 |
| Na Polyphosphate (%) | 0.2 | 0.2 | 0.2 |
| Sodium Nitrite (ppm) | 120 | 120 | 120 |
| Sodium Ascorbate (ppm) | 300 | 300 | 300 |
| Water (%) | 14.5 | 14.5 | 14.5 |
| Peanut Protein (ppm) | 10 | 2 | 0 |
| Total (%) | 99.744 | 99.7422 | 99.742 |

### (4) Confirmation of Detection by Immunochromatography

20 µl of the prepared colloidal gold-labeled antibody, 30 µl of fetal bovine serum (FBS) as a developing solution, and 50 µl of the measurement sample were added, which was tested on an immunochromatographic strip for confirmation of detection.

### 2. Result

Then, the detection results are shown in Table 21. The determination was expressed by +, +w or +- in order of decreasing strength of lines, and negativity was expressed as -.

**[Table 21]**

| <Test Result of Model Meat Product Heated at 75°C for 30 Minutes (Peanut)> | | | | | | | |
|---|---|---|---|---|---|---|---|
| 75°C for 30 Minutes | Sodium Thiosulfate Concentration | | | | | | |
| | 0% | 0.1% | 0.5% | 1.0% | 2.0% | 5.0% | 10.0% |
| 10ppm | + | + | + | + | + | + | +W |
| 2ppm | +W | +W | +W | +W | +W | +W | +- |
| 0ppm | - | - | - | - | - | - | - |

As shown in Table 21, when the concentration of sodium thiosulfate was 0.1% to 10.0%, the protein was detected down to 2 ppm, at which the determination was made as +w particularly at 0.1% to 2.0% and the visibility was best in this range. No non-specific reactions were observed at 0 ppm. This showed that an extraction method in which an anionic surfactant and a thiosulfate were combined was high in the extraction efficiency and eliminated non-specific reactions by using FBS as a developing solution, enabling the construction of a rapid immunochromatography kit with good accuracy.

### Example 12

### [Detection of Peanut Protein Extracted with Anionic Surfactant and Non-Ionic Surfactant by Immunochromatography]

### 1. Materials and Methods

1) Detection was performed in the same way as in Example 11 except that the conditions of the temperature and time of heating model meat products, the pretreatment of samples and the confirmation of detection by immunochromatography were the same as those in Example 2.

### 2. Result

Then, the detection results are shown in Tables 22 and 23. The determination was expressed by +, +w or +- in order of decreasing strength of lines, and negativity was expressed as -.

**[Table 22]**

| <Test Result of Model Meat Product Heated at 75°C for 30 Minutes (Peanut)> | | | |
|---|---|---|---|
| 75°C for 30 Minutes | Model Meat Product | | |
| | 10ppm | 2ppm | 0ppm |
| Measurement Sample [1] | + | + | - |
| Measurement Sample [2] | + | + | - |
| Measurement Sample [3] | + | + | - |

**[Table 23]**

| <Test Result of Model Meat Product Heated at 121°C for 20 Minutes (Peanut)> | | | |
|---|---|---|---|
| 121°C for 20 Minutes | Model Meat Product | | |
| | 10ppm | 2ppm | 0ppm |
| Measurement Sample [1] | + | + | - |
| Measurement Sample [2] | + | + | - |
| Measurement Sample [3] | + | + | - |

As shown in Tables 22 and 23, the determination was made as + down to 2 ppm in all measurement samples, indicating that the peanut protein was able to be detected down to 2 ppm. No non-specific reactions were observed at 0 ppm. Especially, as in the measurement sample [3], the visibility was best with the extraction method in which the anionic surfactant and the non-ionic surfactant were combined. Thus, it was shown that an extraction method in which an anionic surfactant and a non-ionic surfactant were combined was high in the extraction efficiency and eliminated non-specific reactions by using FBS as a developing solution, enabling the construction of a rapid immunochromatography kit with good accuracy.

### Industrial Applicability

There are provided a method for detecting allergens by immunochromatography, capable of rapidly detecting the allergens such as a milk allergen, an egg allergen, a wheat allergen, a buckwheat allergen, and a peanut allergen with good accuracy, and an allergen detection kit for immunochromatography capable of being used for the same.

## Claims

1. A method for detecting allergen by immunochromatography which immunochromatography comprises using:
a colloidal gold-labeled antibody in which a colloidal gold is bound to a monoclonal antibody against denatured and native allergen,
a development support wherein a monoclonal antibody against denatured and native allergen recognizing an epitope different from that recognized by the colloidal gold-labeled antibody is fixed at a predetermined position, and
a developing solution containing a measurement sample of denatured and native allergen extracted from a test sample such as food containing an allergen, using an anionic surfactant and thiosulfate or an anionic surfactant and a non-ionic surfactant;
performing development on the development support;
and detecting the allergen based on the presence of deposition of the colloidal gold;
wherein a developing solution containing at least 10% by weight of fetal bovine serum (FBS) is used.

2. The method for detecting an allergen by immunochromatography according to claim 1, wherein a developing solution containing at least 30% by weight of fetal bovine serum (FBS) is used.

3. The method for detecting an allergen by immunochromatography according to claim 2, wherein a developing solution containing at least 50% by weight of fetal bovine serum (FBS) is used.

4. The method for detecting an allergen by immunochromatography according to any one of claims 1 to 3, wherein the monoclonal antibody against denatured and native allergen are 2 types of monoclonal antibodies specifically recognizing denatured and native allergens selected from αs1 casein as a major component of milk allergen, β-lactoglobulin as a major component of whey allergen, ovalbumin and ovomucoid as egg white allergens, gliadin as a main component of wheat allergen, proteins having molecular weights of 24 kDa and 76 kDa as main proteins of buckwheat, and Arah1 as a main protein of peanut.

5. The method for detecting an allergen by immunochromatography according to any one of claims 1 to 4, wherein dodecyl sodium sulfate is used as anionic surfactant.

6. The method for detecting an allergen by immunochromatography according to any one of claims 1 to 5, wherein Tween 20 is used as non-ionic surfactant.

7. A kit for detecting allergen by immunochromatography comprising: a colloidal gold-labeled antibody in which a colloidal gold is bound to a monoclonal antibody against denatured and native allergen; a development support wherein a monoclonal antibody against denatured and native allergen recognizing an epitope different from that recognized by the colloidal gold-labeled antibody is fixed at a predetermined position; a buffer solution containing an anionic surfactant and thiosulfate or an anionic surfactant and a non-ionic surfactant for extracting denatured and native allergen from a test sample such as food containing an allergen; a carrier for the sample capable of carrying a measurement sample of denatured and native allergen extracted from the test sample such as food containing the allergen with the anionic surfactant and thiosulfate or the anionic surfactant and the non-ionic surfactant; and fetal bovine serum (FBS) or a developing solution containing fetal bovine serum (FBS).

8. The kit for detecting allergen by immunochromatography according to claim 7, wherein the monoclonal antibody against denatured and native allergen are 2 types of monoclonal antibodies specifically recognizing denatured and native allergens selected from αs1 casein as a major component of milk allergen, β-lactoglobulin as a major component of whey allergen, ovalbumin and ovomucoid as egg white allergens, gliadin as a main component of wheat allergen, proteins having molecular weights of 24 kDa and 76 kDa as main proteins of buckwheat, and Arah1 as a main protein of peanut.

9. The kit for detecting allergen by immunochromatography according to claim 7 or 8, comprising dodecyl sodium sulfate as anionic surfactant.

## Patentansprüche

1. Verfahren für den Allergennachweis mittels Immunchromatographie, wobei die Immunchromatographie den Einsatz von Folgendem umfasst:
einem kolloidalen goldmarkierten Antikörper, in dem kolloidales Gold an einen monoklonalen Antikörper gegen denaturiertes und natives Allergen gebunden ist,
einem Entwicklungsträger, wobei ein monoklonaler Antikörper gegen denaturiertes und natives Allergen, der ein Epitop erkennt, das sich von demjenigen, das von dem kolloidalen goldmarkierten Antikörper erkannt wird, unterscheidet, an einer vorbestimmten Position fixiert ist, und
einer Entwicklungslösung, die eine Messprobe von denaturiertem und nativem Allergen enthält, das aus einer Testprobe, wie einem Nahrungsmittel, die ein Allergen enthält, unter Einsatz eines anionischen Tensids und Thiosulfats oder eines anionischen Tensids und eines nichtionischen Tensids extrahiert wurde;
Durchführen der Entwicklung auf dem Entwicklungsträger;
und Nachweisen des Allergens aufgrund des Vorliegens einer Abscheidung des kolloidalen Golds;
wobei eine Entwicklungslösung, die mindestens 10 Gew.-% fötales Rinderserum (FRS) enthält, eingesetzt wird.

2. Verfahren zum Nachweisen eines Allergens mittels Immunchromatographie nach Anspruch 1, wobei eine Entwicklungslösung, die mindestens 30 Gew.-% fötales Rinderserum (FRS) eingesetzt wird.

3. Verfahren zum Nachweisen eines Allergens mittels Immunchromatographie nach Anspruch 2, wobei eine Entwicklungslösung, die mindestens 50 Gew.-% fötales Rinderserum (FRS) enthält, eingesetzt wird.

4. Verfahren zum Nachweisen eines Allergens mittels Immunchromatographie nach einem der Ansprüche 1 bis 3, wobei der monoklonale Antikörper gegen denaturiertes und natives Allergen zwei Arten von monoklonalen Antikörpern darstellt, die spezifisch denaturierte und native Allergene, ausgewählt aus der Reihe αs1-Casein als Hauptkomponente von Milchallergen, β-Lactoglobulin als Hauptkomponente von Molkeallergen, Ovalbumin und Ovomucoid als Eiweißallergene, Gliadin als Hauptkomponente von Weizenallergen, Proteine mit Molekulargewichten von 24 kDa und 76 kDa als Hauptproteine des Buchweizens sowie Arah1 als Hauptprotein der Erdnuss, erkennen.

5. Verfahren zum Nachweisen eines Allergens mittels Immunchromatographie nach einem der Ansprüche 1 bis 4, wobei Dodecylnatriumsulfat als anionisches Tensid eingesetzt wird.

6. Verfahren zum Nachweisen eines Allergens mittels Immunchromatographie nach einem der Ansprüche 1 bis 5, wobei Tween 20 als anionisches Tensid eingesetzt wird.

7. Kit für den Allergennachweis mittels Immunchromatographie, das Folgendes umfasst: einen kolloidalen goldmarkierten Antikörper, in dem kolloidales Gold an einen monoklonalen Antikörper gegen denaturiertes und natives Allergen gebunden ist; einen Entwicklungsträger, wobei ein monoklonaler Antikörper gegen denaturiertes und natives Allergen, der ein Epitop erkennt, das sich von demjenigen, das von dem kolloidalen goldmarkierten Antikörper erkannt wird, unterscheidet, an einer vorbestimmten Position fixiert ist; eine Pufferlösung, die ein anionisches Tensid und Thiosulfat oder ein anionisches Tensid und ein nichtionisches Tensid enthält, zum Extrahieren von denaturiertem und nativem Allergen aus einer Testprobe, wie einem Nahrungsmittel, das ein Allergen enthält; einen Träger für die Probe, der fähig ist, eine Messprobe von denaturiertem und nativem Allergen, das aus der Testprobe, wie einem Nahrungsmittel, die das Allergen enthält, mit dem anionischen Tensid und Thiosulfat oder dem anionischen Tensid und dem nichtionischen Tensid extrahiert worden ist, zu tragen; und fötales Rinderserum (FRS) oder eine Entwicklungslösung, die fötales Rinderserum (FRS) enthält.

8. Kit für den Allergennachweis mittels Immunchromatographie nach Anspruch 7, wobei der monoklonale Antikörper gegen denaturiertes und natives Allergen zwei Arten von monoklonalen Antikörpern darstellt, die spezifisch denaturierte und native Allergene, ausgewählt aus der Reihe αs1-Casein als Hauptkomponente von Milchallergen, β-Lactoglobulin als Hauptkomponente von Molkeallergen, Ovalbumin und Ovomucoid als Eiweißallergene, Gliadin als Hauptkomponente von Weizenallergen, Proteine mit Molekulargewichten von 24 kDa und 76 kDa als Hauptproteine des Buchweizens sowie Arah1 als Hauptprotein der Erdnuss, erkennen.

9. Kit für den Allergennachweis mittels Immunchromatographie nach Anspruch 7 oder 8, das Dodecylnatriumsulfat als anionisches Tensid umfasst.

## Revendications

1. Méthode de détection d'un allergène par immunochromatographie, ladite immunochromatographie comprenant l'utilisation :
d'un anticorps marqué à l'or colloïdal, l'or colloïdal étant lié à un anticorps monoclonal dirigé contre l'allergène dénaturé et natif,
d'un support de révélation où un anticorps monoclonal contre l'allergène dénaturé et natif reconnaissant un épitope différent de celui reconnu par l'anticorps marqué à l'or colloïdal est fixé en une position prédéterminée, et
d'une solution de révélation contenant un échantillon à doser de l'allergène dénaturé et natif extrait d'un échantillon à tester tel qu'un aliment contenant un allergène, en utilisant un tensioactif anionique et du thiosulfate ou un tensioactif anionique et un tensioactif non ionique ;
la mise en oeuvre de la révélation sur le support de révélation ;
et la détection de l'allergène sur la base de la présence de dépôt de l'or colloïdal ;
une solution de révélation contenant au moins 10 % en poids de sérum foetal bovin (SFB) étant utilisée.

2. Méthode de détection d'un allergène par immunochromatographie selon la revendication 1, **caractérisée en ce qu'**on utilise une solution de révélation contenant au moins 30 % en poids de sérum foetal bovin (SFB).

3. Méthode de détection d'un allergène par immunochromatographie selon la revendication 2, **caractérisée en ce qu'**on utilise une solution de révélation contenant au moins 50 % en poids de sérum foetal bovin (SFB).

4. Méthode de détection d'un allergène par immunochromatographie selon l'une quelconque des revendications 1 à 3, **caractérisée en ce que** l'anticorps monoclonal dirigé contre l'allergène dénaturé et natif est constitué de 2 types d'anticorps monoclonaux qui reconnaissent spécifiquement des allergènes dénaturés et natifs choisis parmi la caséine αs1 en tant que composant majeur de l'allergène du lait, la β-lactoglobuline en tant que composant majeur de l'allergène du lactosérum, l'ovalbumine et l'ovomucoïde en tant qu'allergènes du blanc d'oeuf, la gliadine en tant que composant majeur de l'allergène du blé, des protéines ayant des poids moléculaires de 24 kDa et de 76 kDa en tant que protéines principales du sarrasin, et Arah1 en tant que protéine principale de l'arachide.

5. Méthode de détection d'un allergène par immunochromatographie selon l'une quelconque des revendications 1 à 4, **caractérisée en ce qu'**on utilise le dodécylsulfate de sodium comme tensioactif anionique.

6. Méthode de détection d'un allergène par immunochromatographie selon l'une quelconque des revendications 1 à 5, **caractérisée en ce qu'**on utilise Tween 20 en tant que tensioactif non ionique.

7. Kit de détection d'allergène par immunochromatographie comprenant : un anticorps marqué à l'or colloïdal, l'or colloïdal étant lié à un anticorps monoclonal dirigé contre l'allergène dénaturé et natif ; un support de révélation où un anticorps monoclonal dirigé contre l'allergène dénaturé et natif reconnaissant un épitope différent de celui reconnu par l'anticorps marqué à l'or colloïdal est fixé en une position prédéterminée ; une solution tampon contenant un tensioactif anionique et du thiosulfate ou un tensioactif anionique et un tensioactif non ionique pour l'extraction de l'allergène dénaturé et natif d'un échantillon à tester tel qu'un aliment contenant un allergène ; un support pour l'échantillon capable de porter un échantillon à doser de l'allergène dénaturé et natif extrait de l'échantillon à tester tel qu'un aliment contenant l'allergène avec le tensioactif anionique et du thiosulfate ou le tensioactif anionique et le tensioactif non ionique ; et du sérum foetal bovin (SFB) ou une solution de révélation contenant du sérum foetal bovin (SFB).

8. Kit de détection d'allergène par immunochromatographie selon la revendication 7, **caractérisé en ce que** l'anticorps monoclonal dirigé contre l'allergène dénaturé et natif est constitué de 2 types d'anticorps monoclonaux qui reconnaissent spécifiquement des allergènes dénaturés et natifs choisis parmi la caséine αs1 en tant que composant majeur de l'allergène du lait, la β-lactoglobuline en tant que composant majeur de l'allergène du lactosérum, l'ovalbumine et l'ovomucoïde en tant qu'allergènes du blanc d'oeuf, la gliadine en tant que composant majeur de l'allergène du blé, des protéines ayant des poids moléculaires de 24 kDa et de 76 kDa en tant que protéines principales du sarrasin, et Arah1 en tant que protéine principale de l'arachide.

9. Kit de détection d'allergène par immunochromatographie selon la revendication 7 ou 8, comprenant le dodécylsulfate de sodium en tant que tensioactif anionique.
